(19) Europäisches Patentamt
European Patent Office
Office européen des brevets

(11) Veröffentlichungsnummer: **0 176 482**
**B1**

(12) # EUROPÄISCHE PATENTSCHRIFT

(45) Veröffentlichungstag der Patentschrift: **23.01.91**

(51) Int. Cl.[5]: **C 08 G 59/04**

(21) Anmeldenummer: **85810425.0**

(22) Anmeldetag: **18.09.85**

(54) **Verfahren zur Verminderung des Chlorgehaltes in Glycidylverbindungen.**

(30) Priorität: **24.09.84 CH 4556/84**

(43) Veröffentlichungstag der Anmeldung:
**02.04.86 Patentblatt 86/14**

(45) Bekanntmachung des Hinweises auf die Patenterteilung:
**23.01.91 Patentblatt 91/04**

(84) Benannte Vertragsstaaten:
**CH DE FR GB IT LI NL**

(56) Entgegenhaltungen:
**DD-A- 204 483**

**SYNTHESIS, Band 10, Oktober 1970, Stuttgart-London-New York**
**H. KUIVILA "Reduction of Organic Compounds by Organotin Hydrides", Seiten 499-509**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Pallie, Kemal Deen, Dr.**
**Outre-Vièze**
**CH-1871 Choex (CH)**
Erfinder: **Dessauges, Gerald, Dr.**
**Ruelle du Chêne 1**
**CH-1820 Montreux (CH)**

**EP 0 176 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Verminderung des Chlorgehaltes in Glycidylverbindungen durch Reaktion mit organischen Zinnhydriden in Gegenwart von freie Radikale bildenden Initiatoren.

Bekanntlich sind die mittels Epichlorhydrin hergestellten Glycidylverbindungen, insbesondere die technisch hergestellten, stets mit Chlor verunreinigt, das im Epoxidharz als ionisches Chlor und in der Glycidylverbindung als hydrolysierbares (1,2-Chlorhydrin) und als nichthydrolysierbares Chlor (Chlormethyl) vorliegt.

An Epoxidharze, insbesondere solche, die zur Herstellung von elektrischen und elektronischen Bauteilen Verwendung finden, werden laufend höhere Ansprüche bezüglich Reinheit gestellt, um den Korrosionseinfluss des Restchlorgehaltes auf Substrate, insbesondere Kontaktmetalle, zu mindern.

Es sind bereits viele Methoden zur Entfernung des Restchlorgehaltes aus Epoxidharzen bekannt geworden. Wie im "Handbook of Epoxy Resins" (1967), 4—30, von H. Lee and K. Neville dargelegt wird, sind diese Methoden auch mit Nachteilen verbunden.

Sowohl im GB-Patent 1 278 737 als auch in der DE-Offenlegungsschrift 25 23 696 werden spezifische Verfahren zur Herstellung von Polyglycidyläthern durch zweimalige Dehydrohalogenierung mittels Natronlauge offenbart. Der Anteil an Gesamtchlor in den verfahrensgemäss erhaltenen Polyglycidyläthern ist noch relativ hoch. In der JP—Patentanmeldung Kokai 58-173116 wird vorgeschlagen, den Restchlorgehalt in Epoxidharzen mittels Silbersalzen organischer Säuren zu entfernen. Abgesehen davon, dass es sich hierbei um ein kostspieliges Verfahren handelt, haben eigene Nacharbeitungen ergeben, dass der nach diesem Verfahren erzielte Effekt gering ist.

Aus "Synthesis", No. 10, Oktober 1970, Seite 499—509, ist es ferner bekannt, dass Organozinn-hydride selektiv wirkende Reduktionsmittel darstellen und beispielsweise zur Reduktion von Halogenalkanen, Säurehalogeniden, Aldehyden, Ketonen, Estern und Isocyanaten geeignet sind.

Es wurde nun gefunden, dass man mit bestimmten Organozinn-hydriden den Restchlorgehalt in Glycidylverbindungen verringern kann, ohne dass die Glycidylgruppe dabei angegriffen wird.

Gegenstand der vorliegenden Erfindung ist somit ein Verfahren zur Verminderung des Chlorgehaltes in Glycidylverbindungen, deren Glycidylgruppen an Aethersauerstoff-, N- oder S-Atome gebunden sind, dadurch gekennzeichnet, dass man die in einem halogenfreien inerten organischen Lösungsmittel gelöste Glycidylverbindung mit einem Zinnhydrid der Formel I oder II

$$R_3SnH \quad (I) \qquad \text{oder} \qquad R_2SnH_2 \quad (II),$$

worin jedes R unabhängig voneinander je für ein Alkyl mit 1 bis 4 C-Atomen oder Phenyl steht und wobei man pro 1 Aequivalent des vorhandenen Chlors mindestens 1 Aequivalent des Zinnhydrids der Formel I oder II einsetzt, in Gegenwart einer katalytischen Menge eines freie Radikale bildenden Initiators reagieren lässt und danach die Glycidylverbindung aus der Reaktionslösung isoliert.

Für das erfindungsgemässe Verfahren geeignete Glycidylverbindungen, worin die Glycidylgruppen direkt an Aethersauerstoffatome gebunden sind, stellen die Polyglycidyläther dar, die durch Umsetzung einer mindestens zwei freie alkoholische und/oder phenolische Hydroxylgruppen pro Molekül enthaltenden Verbindung mit Epichlorhydrin, Glycerindichlorhydrin oder β-Methylepichlorhydrin unter alkalischen Bedingungen oder auch in Gegenwart eines sauren Katalysators mit nachfolgender Alkalibehandlung erhältlich sind. Diese Aether lassen sich mit den genannten Glycidylierungsmitteln beispielsweise aus acyclischen Alkoholen, wie Aethylenglycol, Diäthylenglykol und höheren Poly-(oxyäthylen)-glykolen, Propan-1,2-diol und Poly-(oxypropylen)-glykolen, Propan-1,3-diol, Poly-(oxytetramethylen)-glykolen, Pentan-1,5-diol, Hexan-2,4,6-triol, Glycerin, 1,1,1-Trimethylolpropan, Pentaerythrit und Sorbit, aus cycloaliphatischen Alkoholen, wie Resorcit, Chinit, Bis-(4-hydroxycyclohexyl)-methan, 2,2-Bis-(4-hydroxycyclohexyl)-propan und 1,1-Bis-(hydroxymethyl)-cyclohexen-3, und aus Alkoholen mit aromatischen Kernen, wie N,N-Bis-(2-hydroxyäthyl)-anilin und p,p'-Bis-(2-hydroxyäthylamino)-diphenylmethan, herstellen. Man kann sie ferner aus einkernigen Phenolen, wie Resorcin und Hydrochinon, und mehrkernigen Phenolen, wie Bis-(4-hydroxyphenyl)-methan (sonst als Bisphenol F bekannt), 4,4'-Dihydroxydiphenyl 1,1,2,2-Tetrakis-(4-hydroxyphenyl)-methan, 2,2-Bis-(4-hydroxyphenyl)-propan (sonst als Bisphenol A bekannt), und aus durch Umsetzung von Aldehyden, wie Formaldehyd, Acetaldehyd und Benzaldehyd, mit Phenol selbst und durch Alkylgruppen mit jeweils bis zu neun Kohlenstoffatome ringsubstituiertem Phenol, wie 2-Methylphenol und 4-tert.-Butylphenol, gebildeten Novolaken erhalten.

Poly-(N-glycidyl)-verbindungen sind ebenfalls für das erfindungsgemässe Verfahren verwendbar, z.B. N-Glycidylderivate von Aminen, wie Anilin, n-Butylamin, Bis-(4-aminophenyl)-methan und Bis-(4-methylaminophenyl)-methan, Triglycidylisocyanurat sowie N,N'-Diglycidylderivate von cyclischen Alkylenharnstoffen, wie Aethylenharnstoff und 1,3-Propylenharnstoff, und von Hydantoinen, wie 5,5-Dimethylhydantoin.

Auch kann man Poly-(S-glycidyl)-verbindungen einsetzen, z.B. Di-(S-glycidyl)-derivate von Dithiolen, wie Aethan-1,2-dithiol und Bis-(4-mercaptomethylphenyl)-äther, doch sind diese nicht bevorzugt.

In Betracht kommen auch Glycidylverbindungen, in welchen die Glycidylgruppen an verschiedene Heteroatome gebunden sind, beispeilsweise p-(Diglycidylamino)-phenylglycidyläther.

Vorzugsweise setzt man beim erfindungsgemässen Verfahren die Glycidyläther, insbesondere die der mehrkernigen Phenole, ein.

Geeignete halogenfreie, inerte organische Lösungsmittel, die im Molekül keine reaktive Gruppe oder Gruppierung bezüglich der Zinnhydride der Formel I oder II enthalten und die beim erfindungsgemässen Verfahren eingesetzt werden können, sind beispielsweise aliphatische Kohlenwasserstoffe, wie Pentan, Hexan, Heptan oder Oktan, cycloaliphatischen Kohlenwasserstoff, wie Cyclohexan oder Cyclopentan, aromatische Kohlenwasserstoff, wie Benzol, Toluol oder Xylole, sowie aliphatische oder cyclische Aether, wie Diäthyläther, Dioxan oder Tetrahydrofuran.

Die im erfindungsgemässen Verfahren einzusetzenden Zinnhydride der Formel I und II stellen bekannte Verbindungen dar und können nach dem in "Journal of Applied Chemistry", Juli 1957, Seite 366—368, oder in "Synthesis", No. 10, Oktober 1970, Seite 499—509, beschriebenen Verfahren hergestellt werden, indem man beispeilsweise die entsprechenden Zinnchloride, $R_3SnCl$ und $R_2SnCl_2$, worin jedem R die Bedeutung von Formel I bzw. II zukommt, mit $LiAlH_4$ zu den Zinnhydriden der Formel I bzw. II reduziert.

Geeignete Zinnhydride für das erfindungsgemässe Verfahren sind beispeilsweise Triäthylzinnhydrid, Tri-n-propylzinnhydrid, Tri-n-butylzinnhydrid, Triphenylzinnhydrid, Di-n-propylzinnhydrid, Di-n-butylzinnhydrid und Diphenylzinnhydrid.

Vorzugsweise setzt man beim erfindungsgemässen Verfahren die Zinnhydride der Formel I ein, insbesondere Tributylzinnhydrid oder Triäthylzinnhydrid.

Die Menge des beim erfindungsgemässen Vefahren einzusetzenden Zinnhydrids der Formel I bzw. II wird so bemessen, dass mindestens 1 H-Aequivalent des Zinnhydrids pro 1 Aequivalent Chlor vorliegt. Vorteilhafterweise setzt man einen Ueberschuss an Zinnhydrid der Formel I oder II ein, der praktisch unbegrenzt gross sein kann, doch vorzugsweise 1,2—20, insbesondere 1,5—12, Aequivalente Zinnhydrid pro 1 Aequivalent Chlor beträgt.

Als freie Radikale bildende Initiatoren können beim erfindungsgemässen Verfahren die thermisch oder photochemisch aktivierbaren Initiatoren eingesetzt werden. Solche Radikalbildner sind bekannt. Einige Radikalbildner stellen sowohl photoaktivierbare (d.h., durch Bestrahlung ausgelöste Bildung freier Radikale), als auch thermisch aktivierbare Substanzen dar.

Beispiele für geeignete photoaktivierbare Initiatoren sind organische Peroxyde und Hydroperoxyde, α-halogensubstituierten Acetophenone, wie Trichlormethyl-4'-tert.-butylphenylketon, α-hydroxy-α-alkylsubstituierte Acetophenone, wie 2-Hydroxy-2-methyl-1-phenylpropanon-1, Benzoin und dessen Alkyläther (z.B. der n-Butyläther), α-Methylbenzoin, Benzophenone, wie Benzophenon selbst und 4,4'-Bis-(dimethylamino)-benzophenon, O-Alkoxycarbonylderivate eines Oxims des Benzils oder 1-Phenylpropan-1,2-dions, wie Benzil-(O-äthoxycarbonyl)-α-monoxim und 1-Phenylpropan-1,2-dion-2-(O-äthoxycarbonyl)-oxim, Benzilketale, wie Benzildimethylketal, substituierte Thioxanthone, Anthrachinone und Photoredoxysysteme, die aus einem Gemisch eines Phenothiazinfarbstoffs (z.B. Methylenblau) oder eines Chinoxalins (z.B. einem Metallsalz der 2-(m- oder p-Methoxyphenyl)-chinoxalin-6'- oder -7'-sulfonsäure) mit einem Elektronendonator, wie Benzolsulfinsäure oder einer anderen Sulfinsäure oder einem Salz davon, wie dem Natriumsalz, oder einem Arsin, einem Phosphin oder Thioharnstoff, bestehen.

Als thermisch aktivierbare, freie Radikale bildende Initiatoren, also solche, die oberhalb der Zimmertemperatur mit beträchtlicher Geschwindigkeit freie Radikale bilden, eignen sich beispielsweise organische oder anorganische Peroxyde, z.B. Persäuren und deren Salze und Ester, wie Peressigsäure, Perbenzoesäure, Perphthalsäure, Diisopropyl-peroxydicarbonat, Ammonium- oder ein Alkaliperborat, Ammonium- oder ein Alkalipersulfat, Acylperoxyde, wie Benzoylperoxyd, sowie z.B. Cumylperoxyd, Cumolhydroperoxyd, Wasserstoffperoxyd, Cyclohexanonperoxyd und Aethylmethylketonperoxyd, Azoverbindungen, wie Azo-bis-(isobutyronitril), und sterisch gehinderte phenyl-substituierte Alkane, wie 2,3-Dimethyl-diphenylbutan und 3,4-Dimethyl-3,4-diphenylhexan. Als Radikalinitiatoren werden die thermisch aktivierbaren, wie Azo-bis-(isobutyronitril), Cumolhydroperoxid und Ketonperoxide, besonders Azo-bis-(isobutyronitril), bevorzugt.

Die freie Radikale bildenden Initiatoren werden beim erfindungsgemässen Verfahren üblicherweise in katalytischen Mengen eingesetzt, d.h., ihr Anteil beträgt im allgemeinen zwischen 0,1 bis 5 Gewichts-%, bezogen auf die Menge der eingesetzten Glycidylverbindung, vorzugsweise zwischen 0,1 bis 1,5 Gewichts-%.

Das erfindungsgemässe Verfahren wird bevorzugt bei erhöhten Temperaturen durchgeführt, insbesondere im Temperaturbereich von 60 bis 110°C.

Die Aufarbeitung des Reaktionsgemisches kann nach üblichen Trennungsmethoden vorgenommen werden. Beispielsweise kann man nach dem Entfernen des organischen Lösungsmittels das überschüssige Zinnhydrid der Formel I oder II und das bei der Reaktion gebildete Alkyl- bzw. Phenylzinnchlorid entweder unter vermindertem Druck destillativ entfernen oder man löst nach Entfernen des organischen Lösungsmittels die zurückbleibende Glycidylverbindung in Acetonitril und wäscht diese Lösung zwecks Entfernen der organischen Zinnverbindungen mit Hexan oder Petroläther aus. Die letztere Methode stellt eine bevorzugte Aufarbeitungsweise dar.

Die in den folgenden Beispielen angegebenen Mengen an Gesamtchlor und hydrolysierbarem Chlor werden potentiometrisch wie folgt bestimmt:

Gesamtchlorgehalt: Zu etwa 3 g der Glycidylverbindung werden 25 ml Butylcarbitol (Diäthylenglykolmonobutyläther) zugegeben. Die Lösung wird 20 Minuten unter Rückfluss erhitzt und anschliessend auf Zimmertemperatur abgekühlt. Nach Zugabe von 50 ml Essigsäure wird der Chlorgehalt potentiometrisch mittels einer 0,01 n Silbernitratlösung bestimmt.

Gehalt an hydrolysierbarem Chlor: Etwa 5 g der Glycidylverbindung werden in 20 ml Toluol gelöst und mit 50 ml einer 0,1 n Kalilaugelösung versehen. Die Lösung wird 2 Minuten unter Rückfluss erhitzt und dann auf 10 bis 15°C gekühlt. Nach Zugabe von 50 ml Essigsäure wird der Chlorgehalt potentiometrisch mittels einer 0,01 n Silbernitratlösung bestimmt.

Wie schon erwähnt, sind die nach dem erfindungsgemässen Verfahren erhaltenen Glycidylverbindungen besonders für Anwendungen auf dem Elektroniksektor geeignet.

Beispiel 1:

101 g eines aus Bisphenol A und Epichlorhydrin technisch hergestellten Bisphenol A-diglycidyläthers mit einem Epoxidgehalt von 5,37 Aequivalenten/kg, einem Gesamtchlorgehalt von 0,16 Gewichts-% und einem Gehalt an hydrolysierbarem Chlor von 0,024 Gewichts-% werden in 200 g Toluol gelöst. Die Lösung wird auf 80°C erhitzt und unter $N_2$-Atmosphäre gibt man 7,5 g Tri-n-butylzinnhydrid und 0,2 g Azobis-(isobutyronitril) hinzu. Nach dem Rühren der Lösung während 16 Stunden bei dieser Temperatur wird das Toluol mittels Destillation entfernt und das zurückbleibende Epoxidharz in 100 g Acetonitril gelöst. Aus dieser Lösung wird das bei der Reaktion entstandene Tributylzinnchlorid und das überschüssige Tributylzinnhydrid durch 10 maliges Waschen mit je 100 g Hexan entfernt. Der nach dem Abdestillieren des Acetonitrils erhaltene Bisphenol A-diglycidyläther weist einen Epoxidgehalt von 5,36 Aequivalenten/kg, einen Gesamtchlorgehalt von 0,033 Gewichts-% und einen Gehalt von 0,009 Gewichts-% an hydrolysierbarem Chlor auf.

Beispiel 2:

100 g eines aus Kresolnovolak und Epichlorhydrin technisch hergestellten Kresolnovolakepoxidharzes mit einem Epoxidgehalt von 4,62 Aequivalenten/kg, einem Gesamtchlorgehalt von 0,13 Gewichts-% und einem Gehalt an hydrolysierbarem Chlor von 0,028 Gewichts-% werden in 180 g Toluol gelöst. Die Lösung wird auf 80°C erhitzt und unter $N_2$-Atmosphäre gibt man 4,18 g Tri-n-butylzinnhydrid und 0,2 g Azo-bis-(isobutyronitril) hinzu. Die Reaktionslösung wird auf 80°C gehalten, und nach 1 Stunde werden erneut 0,1 g Azo-bis-(isobutyronitril) zur Reaktionslösung zugegeben. Dieser Vorgang wird 3 mal wiederholt. Nach einer gesamten Reaktionsdauer von 20 Stunden bei 80°C wird die Reaktionslösung wie im Beispiel 1 aufgearbeitet. Man erhält ein Kresolnovolakepoxidharz mit einem Epoxidgehalt von 4,6 Aequivalenten/kg, einen Gesamtchlorgehalt von 0,032 Gewichts-% und einem Gehalt an hydrolysierbarem Chlor von 0,0084 Gewichts-%.

Beispiel 3:

100 g des in Beispiel 2 eingesetzten Kresolnovolakepoxidharzes werden in 200 g Toluol gelöst. Die Lösung wird auf 80°C erhitzt und unter $N_2$-Atmosphäre gibt man 4,5 g Triäthylzinnhydrid und 0,1 g Azo-bis-(isobutyronitril) hinzu. Die Lösung wird bie 80°C gehalten und nach 1 Stunde gibt man erneut 0,1 g Azo-bis-(isobutyronitril) hinzu. Dieser Vorgang wird 3 mal wiederholt. Nach einer gesamten Reaktionszeit von 22 Stunden bei 80°C wird das Toluol abdestilliert und das Zinnsalz durch Strippen mit Wasser aus dem Epoxidharz entfernt. Man erhält ein Kresolnovolakepoxidharz mit einem Epoxidgehalt von 4,56 Aequivalenten/kg, einem Gesamtchlorgehalt von 0,037 Gewichts-% und einem Gehalt an hydrolysierbarem Chlor von 0,02 Gewichts-%.

Beispeil 4:

100 g des in Beispiel 1 eingesetzten Bisphenol A-diglycidyläthers werden in 200 g Toluol gelöst. Die Lösung wird auf 80°C erhitzt und unter Stickstoffatmosphäre werden 2,89 Triäthylzinnhydrid und 0,1 g Azo-bis-(isobutyronitril) zugegeben. Die Lösung wird auf 80°C gehalten und nach 1 Stunde gibt man erneut 0,1 g Azo-bis-(isobutyronitril) hinzu. Dieser Vorgang wird 3 mal wiederholt. Nach einer Gesamtreaktionszeit von 22 Stunden bei 80°C wird das Toluol abestilliert und die restlichen Zinnverbindungen mittels Vakuumdestillation bei 160°C/130 Pa aus dem Epoxidharz entfernt. Man erhält einen Bisphenol A-diglycidyläther mit einem Epoxidgehalt von 5,24 Aequivalenten/kg, einem Gesamtchlorgehalt von 0,0403 Gewichts-% und einem Gehalt an hydrolysierbarem Chlor von 0,033 Gewichts-%.

Beispiel 5:

100 g des in Beispiel 2 eingesetzten Kresolnovolakepoxidharzes werden in 100 g Toluol gelöst und unter $N_2$-Atmosphäre gibt man 0,62 g Azo-bis-(isobutyronitril) und 3,3 g Di-n-butylzinnhydrid hinzu. Die Lösung wird auf 80°C erhitzt und 4 Stunden bei dieser Temperatur gehalten. Dann wird die Reaktionslösung wie im Beispiel 1 beschrieben aufgearbeitet. Man erhält ein Kresolnovolakepoxidharz mit einem Epoxidgehalt von 4,6 Aequivalenten/kg, einem Gesamtchlorgehalt von 0,067 Gewicht-% und einem Gehalt an hydrolysierbarem Chlor von 0,0097 Gewichts-%.

Beispiel 6:

100 g des in Beispiel 2 eingesetzten Kresolnovolakepoxidharzes werden in 100 g Toluol gelöst und unter $N_2$-Atmosphäre gibt man 0,62 g Azo-bis-(isobutyronitril) und 5,79 g Triphenylzinnhydrid hinzu. Die lösung wird auf 80°C erhitzt und 4 Stunden bei dieser Temperatur gehalten. Dann wird die Reaktionslösung wie im Beispiel 1 beschrieben aufgearbeitet. Man erhält ein Kresolnovolakepoxidharz mit einem Epoxidgehalt von 4,43 Aequivalenten/kg, einem Gesamtchlorgehalt von 0,0255 Gewicht-% und einem Gehalt an hydrolysierbarem Chlor von 0,0053 Gewichts-%.

## Patentansprüche

1. Verfahren zur Verminderung des Chlorgehaltes in Glycidylverbindungen, deren Glycidylgruppen an Aethersauerstoff-, N- oder S-Atome gebunden sind, dadurch gekennzeichnet, dass man die in einem halogenfreien, inerten organischen Lösungsmittel gelöste Glycidylverbindung mit einem Zinnhydrid der Formel I oder II

$$R_3SnH \quad (I) \qquad \text{oder} \qquad R_2SnH_2 \quad (II),$$

worin jedes R unabhängig voneinander je für ein Alkyl mit 1 bis 4 C-Atomen oder Phenyl steht und wobei man pro 1 Aequivalent des vorhandenen Chlors mindestens 1 Aequivalent des Zinnhydrids der Formel I oder II einsetzt, in Gegenwart einer katalytischen Menge eines freie Radikale bildenden Initiators reagieren lässt und danach die Glycidylverbindung aus der Reaktionslösung isoliert.

2. Verfahren gemäss Anspruch 1, dadurch gekennzeichnet, dass man als Glycidylverbindung einen Glycidyläther einsetzt.

3. Verfahren gemäss Anspruch 1, dadurch gekennzeichnet, dass man als Glycidylverbindung einen Glycidyläther eines mehrkernigen Phenols einsetzt.

4. Verfahren gemäss Anspruch 1, dadurch gekennzeichnet, dass man als Zinnhydrid solche der Formel I einsetzt.

5. Verfahren gemäss Anspruch 1, dadurch gekennzeichnet, dass man als Zinnhydrid der Formel I Tri-n-butylzinnhydrid, Triäthylzinnhydrid oder Triphenylzinnhydrid einsetzt.

6. Verfahren gemäss Anspruch 1, dadurch gekennzeichnet, dass man pro 1 Aequivalent Chlor 1,2 bis 20 Aequivalente Zinnhydrid der Formel I oder II einsetzt.

7. Verfahren gemäss Anspruch 1, dadurch gekennzeichnet, dass man pro 1 Aequivalent Chlor 1,5 bis 12 Aequivalente Zinnhydrid der Formel I oder II einsetzt.

8. Verfahren gemäss Anspruch 1, dadurch gekennzeichnet, dass man als Initiator einen thermisch aktivierbaren freie Radikale bildenden Initiatoren einsetzt.

9. Verfahren gemäss Anspruch 1 oder 8, dadurch gekennzeichnet, dass man als Initiator Azo-bis-(isobutyronitril) einsetzt.

10. Verfahren gemäss Anspruch 1, dadurch gekennzeichnet, dass man die Reaktion im Temperaturbereich von 60 bis 110°C durchführt.

11. Verfahren gemäss Anspruch 1, dadurch gekennzeichnet, dass man nach der Reaktion die Glycidylverbindung vom organischen Lösungsmittel befreit, in Acetonitril löst und diese Lösung mit Hexan oder Petroläther wäscht.

## Revendications

1. Procédé pour diminuer la teneur en chlore de composés glycidyliques, dont les groupes glycidyles sont liés à des atomes d'oxygène de fonction éther-oxyde, à des atomes de N ou de S, procédé caractérisé en ce qu'on soumet le composé glycidylique, dissous dans un solvant organique inerte sans halogène, à une réaction avec un hydrure d'étain de formules I ou II:

$$R_3SnH \quad (I) \qquad \text{ou} \qquad R_2SnH_2 \quad (II),$$

(dans lesquelles chaque symbole R représente, indépendamment l'un de l'autre, un groupe alkyle ayant 1 à 4 atomes de carbone ou un groupe phényle), en utilisant, pour 1 équivalent du chlore présent, au moins 1 équivalent de l'hydrure d'étain de formules I ou II, en présence d'une quantité catalytique d'un amorceur engendrant des radicaux libres, puis l'on isole le composé glycidylique de la solution de réaction.

2. Procédé selon la revendication 1, caractérisé en ce qu'on utilise, comme composé glycidylique, un éther-oxyde de glycidyle.

3. Procédé selon la revendication 1, caractérisé en ce qu'on utilise comme composé glycidylique un étheroxyde glycidylique d'un phénol polycyclique.

4. Procédé selon la revendication 1, caractérisé en ce qu'on utilise, comme hydrure d'étain, ceux répondant à la formule I.

5. Procédé selon la revendication 1, caractérisé en ce qu'on utilise comme hydrure d'étain de formule I l'hydrure de tri-butyl-étain, l'hydrure de tri-éthyl-étain ou l'hydrure de triphényl-étain.

6. Procédé selon la revendication 1, caractérisé en ce qu'on utilise, pour 1 équivalent de chlore, 1,2 à 20 équivalents d'hydrure d'étain de formules I ou II.

7. Procédé selon la revendication 1, caractérisé en ce qu'on utilise pour 1 équivalent de chlore 1,5 à 12 équivalents d'hydrure d'étain de formules I ou II.

8. Procédé selon la revendication 1, caractérisé en ce qu'on utilise, comme amorceur, un amorceur formant des radicaux libres et activable par voie thermique.

9. Procédé selon la revendication 1 ou 8, caractérisé en ce qu'on utilise comme amorceur l'azo-bis-(isobutyronitrile).

10. Procédé selon la revendication 1, caractérisé en ce qu'on conduit la réaction dans le domaine de température allant de 60 à 110°C.

11. Procédé selon la revendication 1, caractérisé en ce qu'après la réaction, on élimine le solvant organique du composé glycidylique, on dissout celui-ci dans de l'acétonitrile et on lave cette solution avec de l'hexane ou de l'éther de pétrole.

## Claims

1. A process for reducing the content of chlorine in a glycidyl compound in which the glycidyl groups are bonded to ether-oxygen, N or S atoms, which comprises reacting the glycidyl compound, dissolved in a halogen-free inert organic solvent, with a tin hydride of the formula I or II

$$R_3SnH \quad (I) \qquad or \qquad R_2SnH_2 \quad (II)$$

in which each radial R independently of one another is an alkyl of 1 to 4 C atoms or phenyl, at least 1 equivalent of the tin hydride of the formula I or II being employed per equivalent of chlorine present, in the presence of a catalytic amount of an initiator which forms free radicals, and then isolating the glycidyl compound from the reaction solution.

2. The process according to Claim 1, wherein a glycidyl ether is employed as the glycidyl compound.

3. The process according to Claim 1, wherein a glycidyl ether of a polynuclear phenol is employed as the glycidyl compound.

4. The process according to Claim 1, wherein a tin hydride of the formula I is employed as the tin hydride.

5. The process according to Claim 1, wherein tri-n-butyl-tin hydride, triethyl-tin hydride or triphehyl-tin hydride is employed as the tin hydride of the formula I.

6. The process accogding to Claim 1, wherein 1.2 to 20 equivalents of tin hydride of the formula I or II are employed per equivalent of chlorine.

7. The process according to Claim 1, wherein 1.5 to 12 equivalents of tin hydride of the formula I or II are employed per equivalent of chlorine.

8. The process according to Claim 1, wherein an initiator which forms free radicals and can be activated by heat is employed as the initiator.

9. The process according to either of Claims 1 or 8, wherein azo-bis-(isobutyronitrile) is employed as the initiator.

10. The process according to Claim 1, wherein the reaction is carried out in the temperature range from 60 to 110°C.

11. The process according to Claim 1, wherein, after the reaction, the glycidyl compound is freed from the organic solvent and dissolved in acetonitrile and this solution is washed with hexane or petroleum ether.